Europäisches Patentamt

European Patent Office

Office européen des brevets

(11) Publication number: **0 504 435 A1**

## EUROPEAN PATENT APPLICATION
### published in accordance with Art. 158(3) EPC

(21) Application number: **91918153.7**

(22) Date of filing: **09.10.91**

(86) International application number:
**PCT/SU91/00200**

(87) International publication number:
**WO 92/07089 (30.04.92 92/10)**

(51) Int. Cl.5: **C12Q 1/68**

(30) Priority: **10.10.90 SU 4870176**

(43) Date of publication of application:
**23.09.92 Bulletin 92/39**

(84) Designated Contracting States:
**DE GB**

(71) Applicant: **ONISCHENKO, Anatoly Mikhailovich Institutsky gorodok, 9-108, pos. Jurievets**

Vladimir, 600900(SU)

(72) Inventor: **ONISCHENKO, Anatoly Mikhailovich Institutsky gorodok, 9-108, pos. Jurievets Vladimir, 600900(SU)**

(74) Representative: **Rambelli, Paolo et al Jacobacci-Casetta & Perani S.p.A. Via Alfieri 17 I-10121 Torino(IT)**

(54) **METHOD AND DEVICE FOR AMPLIFICATION OF DNA.**

(57) A method for amplification of DNA consists in that a reaction mixture is prepared composed of the matrix DNA, synthetic oligonucleotide primers, desoxynucleosidetriphosphates and a buffer medium. The process is carried out in a continuous closed system consisting of the following thermoregulated zones: a DNA melting zone and a cooling zone which provide for denaturation of the matrix DNA; a zone of annealing with the primers and a complementary chain building zone; in the said system, circulation is effected of the prepared reaction mixture, which is a carrier of DNA molecules which, while passing consecutively through each of said zones in the course of circulation, are subjected to denaturation, annealing with the primers and building of the complementary chain; the circulation of the reaction mixture being effected until the desired quantity of the synthesized DNA is obtained. A device for amplification of DNA comprises a closed conduit consisting of capillary tubes each placed in an incubator maintaining the desired temperature conditions for the melting zone, cooling zone, annealing zone and complementary chain building zone.

Field of the Invention

The invention relates to biochemistry and, more specifically, to a method and apparatus for amplification of DNA.

State of the Art

Various methods are known to amplify DNA, and these methods involve adding to a DNA isolated from some organism chemically synthesized oligonucleotides (primers) which are capable of recognizing predetermined DNA sequences and of (complementarily) fusing with them. These complexes are recognized by a DNA-polymerase which completes the end of the oligonucleotide with a sequence complementary to the starting sequence from among nucleotides available in a solution. The process is, therefore, conducted in three phases: denaturation of a template DNA, annealing with primers, and completion of the complementary chain. These phases are then repeated until the process is over (PCR Technology. Principles and Applications for DNA Amplification. ed. Henry A. Erlich. Munchen. Stockton Press. 1989. p. 1-7).

In a known method for amplification of DNA (PCR Technology. Principles and Applications for DNA Amplification. ed. Henry A. Erlich. Munchen. Stockton Press. 1989. 7-16; 23-25) a reaction mixture is prepared to contain 50 mM KCl, 10 mM Tris HCl (pH = 8.4 at room temperature), 1.5 mM $MgCl_2$, 100 $\mu$l gelatin, 250 $\mu$l of each dNTP, 2.5 U of Taq-polymerase, and 1 mM of each primer (oligonucleotide). DNA is used in a starting amount of template between $10^2$ and $10^5$ copies. The reaction mixture volume is 50 to 100 $\mu$l. Template DNA is denatured at t = 94°C during 20 s, and annealing with primers is then carried out at 55°C during 20 s with completion of the complementary chain at 72°C during 30 s. The process is conducted at a heating rate of 0.8°C/s and a cooling rate of 1°C/s. For amplification of, e.g., a DNA fragment of a length between 150 and 3000 base pairs the duration of the cycle is up to 3.75 minutes. The process involves 30 cycles.

The state-of-the art methods have a low yield of the end product or synthetic DNA because of a limited cooling rate. The limited cooling rate results in agglutination of molecules which cannot take part in the process, and it is difficult to control temperature of the process.

Two different types of apparatuses can be used for carrying out the above-described methods (PCR Technology. Principles and Applications for DNA Amplification. ed. Henry A. Erlich. Munchen. Stockton Press. 1989). One type involves the use of a group of thermostats for ensuring various treatment conditions, the reaction mixture being placed into test tubes. The test tubes are transferred between thermostats by means of robots which grip the test tubes are transfer them from one thermostat into another. The main disadvantage of this equipment resides in the fact that temperature conditions are inevitably changed when a test tube is transferred from one thermostat into another. This affects the reaction, hence, quality of the end product. In addition, a change in temperature conditions affects the yield of DNA as well.

The other type of equipment involves the use of a program-controlled thermostat, and various test tubes are placed in one and the same thermostat which is automatically switched over from one temperature mode to another. It should be, however, noted that a change from one temperature mode to another occurs during a comparatively prolonged time so as to affect the reaction, prolong the amplification process and finally affect the yield of DNA. All groups are treated under similar temperature conditions so that the process cannot be optimized and different mixtures cannot be treated simultaneously. If a plurality of program-controlled thermostats are used, the preparation of the end product becomes very expensive because of high cost of such thermostats.

Summary of the Invention

The invention is based on the problem of modifying process steps in such a manner as to increase the yield of the end product and simplify the process and also of providing an apparatus for carrying out the process.

This problem is solved by the fact that in a method for amplification of DNA comprising preparing a reaction mixture consisting of a template DNA, primers of synthetic oligonucleotides, desoxynucleosidetriphosphates, and a buffer medium, carrying out thermocontrolled denaturation of the template DNA, annealing with primers, and completion of the complementary chain, with subsequent repetition of the steps of denaturation, annealing and completion until the process is over, according to the invention, the process is conducted in a continuous closed-loop system consisting of the following thermocontrolled zones: a DNA fusion zone and a cooling zone ensuring denaturation of DNA, a zone for annealing with primers, and a zone for completion of the complementary chain, circulation of the prepared reaction mixture, which is a carrier of DNA molecules, is carried out in this system, the molecules being denatured, annealed with primers, and the complementary chain is completed when they pass through each of said zones during the circulation, the circulation of the reaction mixture being carried out until a predetermined amount of DNA is prepared.

Temperature in the fusion zone is preferably maintained within the range from 80 to 95°C and temperature in the cooling zone is preferably maintained within the range from 10 to 30°C. These temperature ranges ensure fusion of both short and long DNA molecules. They rule out agglutination of molecules and reduce degrading of DNA-polymerase and desoxynucleosidetriphosphates so as to enhance efficiency of denaturation of a template DNA. Temperature in the annealing zone is preferably maintained within the range of 20 to 60°C, and temperature in the annealing zone is preferably maintained within the range of 50 to 75°C. This ensures more accurate and efficient addition of oligonucleotide primers to the template DNA molecule and their strong bond.

According to the invention, circulation is preferably carried out at a reaction mixture heating rate of 0.3 to 0.6°C/s and with a cooling rate of 8 to 20°C/s. This makes the process of DNA denaturation more efficient by ruling out agglutination of molecules. The method according to the invention allows the yield of the end product to be increased by a factor of 2 to 5 in comparison with the state-of-the art methods. The procedure is simplified owing to a modified thermocontrol system. The method allows any preset quantities of synthesized DNA to be produced on commercial scale.

The method according to the invention may be carried out by means of a special apparatus for amplification of DNA which, according to the invention, comprises a closed-loop pipeline adapted to be filled with a fluid reaction mixture. The apparatus is provided with four thermostats mounted downstream one another along the perimeter of the pipeline, the thermostats defining preset temperature zones in the pipeline which correspond in the process sequence to the fusion zone, cooling zone, annealing zone, and a zone for completion of the complementary chain.

This construction of the apparatus allows all advantages of the process according to the invention to be obtained. The use of a closed-loop system of vessels and a zonal arrangement of the thermostats allow special sophisticated devices for transfer of a reaction mixture between various thermostating zones to be dispensed with. As a result, the process is simplified, and the effect of accidental sudden changes in temperature, which are inevitable in prior art devices for amplification of DNA, can be ruled out. Accurate compliance with thermodynamic conditions is thereby ensured so as to contribute to the best conditions of DNA synthesis.

The pipeline may be made in the form of a single ring-shaped tube, but in such case it is technically difficult to place the tube in thermostats.

It is, therefore, preferred that the pipeline be made up of four consecutively communicating tubes, each tube being placed in a respective individual thermostat which ensures desired temperature conditions.

Since amplification of DNA requires uniform heating of the reaction mixture over the whole volume, it is preferred that capillary tubes be used for making up the closed-loop pipeline.

The length of a resulting molecule is determined by a number of factors, among which is the capacity of the closed-loop system. It is, therefore, preferred that the tubes be installed on a common base and be detachably connected to one another so as to facilitate replacement.

The rate of movement of a reaction mixture may be controlled by a speed controller.

Description of the Drawings

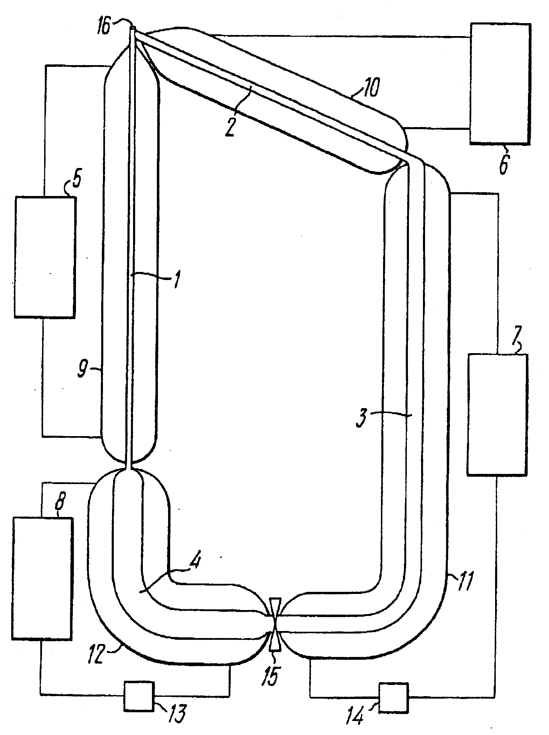

The invention will now be described with reference to specific embodiments of a method for amplification of DNA illustrated in the accompanying drawing which schematically shows an apparatus for carrying out the method according to the invention.

Best Way of Carrying out the Invention

For carrying out a method according to the invention, a reaction mixture is prepared with the use of a conventional procedure. A reaction mixture consists of a template DNA, primers or synthetic oligonucleotides, desoxynucleosidetriphosphates, a DNA-polymerase, and a buffer solution. A template DNA may be in the form of a DNA of any composition (with any ratio of AT to GC pairs) and of any length. The reaction mixture is charged into a continuous closed-loop system which consists of four thermocontrolled zones: a DNA fusion zone, a cooling zone, a zone for annealing with primers, and a zone for completion of the complementary chain. The reaction mixture, which is a carrier of DNA molecules, is caused to circulate in this system. During the circulation DNA molecules, which pass consecutively through each of the above-mentioned zones, are subjected to denaturation, annealing with primers, and completion of the complementary chain. A template DNA is denaturated in the fusion and cooling zones. A temperature in the fusion zone ranges from 80 to 95°C. This temperature range ensures fusion of both short DNA molecules (60 to 80 base pairs) and long molecules of over 1000 base pairs. The temperature conditions in this zone allow fusion of a template DNA to be ensured and rule out agglutination of molecules. Thermal degrading of DNA-polymerase and desoxynucleosidetriphosphates is reduced.

If temperature drops below the fusion point of a DNA molecule, the agglutination process will prevail, and the longer the residence time of DNA molecules within this temperature range, the greater is the amount of molecules sticking to each other so that the yield of the end product decreases. To prevent agglutination of molecules, it is necessary to cool the reaction mixture as fast as possible. This is ensured by the fact that a rate of cooling of the reaction mixture ranges from 8 to 20°C. The cooling zone preferably has a temperature of 10 to 30°C. Therefore, circulation of the reaction mixture at a heating rate of 0.3 to 0.6°C/s and a cooling rate of 8 to 20°C/s as well as the temperature conditions set up for DNA fusion and cooling zones allow denaturation of DNA to be carried out efficiently without molecules sticking together. Temperature conditions for annealing with primers at 20 to 60°C ensures most accurate and efficient addition of oligonucleotide primers to a template DNA molecule. A temperature in the zone of completion of the complementary chain between 50 and 75°C ensures a gradual temperature transition between the annealing zone and the completion zone. The gradual temperature transition is necessary for ensuring a strong bond between primers and a starting template. In the event a sudden temperature increase occurs, a complex consisting of a primer and template fuses, and further synthesis and completion are interrupted to reduce efficiency of amplification and lower the yield of the end product. The reaction mixture is circulated until a predetermined amount of DNA is synthesized.

An embodiment of an apparatus for amplification of DNA and various examples of the method of amplification carried out with the aid of this apparatus will be described below.

An apparatus for amplification of DNA schematically shown in a sole figure of the drawings is built around capillary tubes 1, 2, 3 and 4 and diagrammatically shown thermostats 5, 6, 7 and 8 having chambers 9, 10, 11 and 12, respectively. The capillary tubes communicate in series with one another to define a closed-loop pipeline which is arranged to extend in a vertical plane and which is adapted to be filled with a reaction mixture. Capillary tube 1 is placed in chamber 9 of thermostat 5 which is capable of maintaining temperatures within the range from 80 to 95°C for defining a fusion zone. Capillary tube 2 is placed in chamber 10 of thermostat 6 which is capable of maintaining a temperature ranging from 10 to 30°C to define a cooling zone. Capillary tube 3 is placed in chamber 11 of thermostat 7 which is capable of maintaining a temperature ranging from 20 to 60°C to define a zone for annealing. Capillary tube 4 is placed in chamber 12 of thermostat 8 which is capable of providing temperatures ranging from 20 to 60°C to define a zone for completion of the complementary chain. Portions of capillary tubes 1, 2, 3 and 4 located outside the thermostats should have a reliable thermal protection to avoid the effect of the ambient temperature. Since capillary tubes 3 and 4 are of a large capacity and intermixing of layers can occur in these tubes, thermostats 7 and 8 are provided with devices 13 and 14 for creating a temperature gradient lengthwise of these capillary tubes to rule out intermixing of layers and improve conditions for preparing the end product. A change in the velocity of movement of a reaction mixture through the closed-loop pipeline can be ensured by means of a speed controller 15, e.g., in the form of a variable throttle. Loading of a reaction mixture and discharge of the end product are carried out through a port 16 which can be stopped by means of a liquid stopper allowing gas removal from the reaction mixture.

Example 1

A starting reaction mixture was prepared to have the following composition: 10 $\mu$l of a buffer solution containing 500 mM KCl, 100 mM Tris-HCl (pH = 8.4), 15 mM, 15 mM MgCl$^2$, 0.1 $\mu$g/ml BSA, 1% Np-40. 100 pmoles of each of the following primers were then added:

(1) primer of identical sequence ovalbumin m-RNA at position of 223-250 bases of the following primary structure:

5'-GGCACATGTCTAAACGATCACTCTTCAC-3';

(2) primer of the complementary sequence of ovalbumin m-RNA at a position of 651-587 bases of the following primary structure:

5'-GCTTGTGTGTCTTGATCCTTAAATGC-3'

with final concentration of each of them 1 mM, cDNA gene of ovalbumin in an amount of 20 pg. dNTR (2.5 mM) in an amount of 10 $\mu$l, 2 U of Taq-polymerase and water to 100 ml were then added.

The resultant reaction mixture was poured to fill the closed-loop system formed by series-connected vessels in the form of capillary tubes each placed in an individual thermostat. Vaseline oil was poured in layers into port 16 to seal the apparatus.

The thermostats were heated to obtain the following working modes: thermostat 6 to 15°C, thermostat 7 to 42°C, thermostat 8 to 68°C, and thermostat 5 to 95°C.

The reaction mixture circulated on its own under the action of the above-mentioned temperature conditions in the closed-loop system to function as a carrier for DNA molecules which passed consecutively through each of the above-mentioned temperature zones to be denaturated, annealed with primers and for completion of the complementary chain. The rate of heating of the reaction mixture during denaturation was 6.0°C/s and the rate of cooling was 8.5°C.

Speed controller 15 was used for a cycle of 3.75 minutes, and 30 cycles were conducted.

The end product was removed through port 16. The synthesized DNA were in the form of a gene of ovalbumin with a length of 364 base pairs.

A process was also carried out with the use of a conventional procedure with a similar starting reaction mixture. The cycle time was 3.75 minutes, and 30 cycles were conducted.

Comparison of the end products prepared according to the invention and with the use of the conventional technique (R. K. Saike) was carried out. Each product (15 $\mu$l) were applied to a polyarylamide gel. The resulting electrophoretic chart was subjected to densitometry. The densitometry showed that concentration of the prepared substance was three times as great as concentration of the material amplified by using the prior art technique.

Example 2

A starting reaction mixture was prepared to have the following composition: 10 $\mu$l of a buffer solution containing 500 mM KCl, 100 mM Tris-HCl (pH = 8.4), 15 mM MgCl$_2$, 0.1 $\mu$g/ml BSA, 1% Np-40, 100 pmoles of each of the following primers:

(1) primer of identical sequence of ovalbumin m-RNA at position of 913-929 bases of the following primary structure:

5'-CTGATCTGTTTAGCTCTTC-3';

(2) primer of the complementary sequence of ovalbumin m-RNA in the area of 1056-1072 bases of the following primary structure:

5'-GATCACAGACGGTTGAT-3',

with the final concentration of each being 1 mM, 20 pg of cDNA of ovalbumin gene. dTTP (2.5 mM) in an amount of 10$\mu$l, 2 U of Taq-polymerase, and water to 100 $\mu$l were then added.

The closed-loop system defined by series communicating vessels in the form of capillary tubes each placed in an individual thermostat was then filled with the resulting reaction mixture. Vaseline oil (30 $\mu$l) was poured in layers into port 16 to seal the apparatus.

The thermostats were heated to obtain the following working modes: thermostat 6 to 15°C; thermostat 7 with a gradient from 37 to 42°C; thermostat 8 to a temperature from 55°C to 68°C, and thermostat 5 to 95°C.

The reaction mixture circulated on its own under the action of these temperature conditions through the closed-loop system to function as a carrier of DNA molecules to pass consecutively through each of the above-mentioned temperatures zones to be subjected to denaturation and annealing with primers and for completion of the complementary chain.

The cooling rate was 8.5°C/s and the heating rate was 0.3°C/s. The flow velocity was set up in such a manner that the cycle time was 2.5 minutes and 30 cycles were carried out.

The end product was removed from port 16. The synthesized DNA were in the form of fragments of ovalbumin gene of a length of 159 base pairs.

A process was also carried out by using the conventional technique with a similar reaction mixture. Thirty 2.5-minute cycles were carried out. Comparison between the end products according to the invention and those produced by conventional procedure (R. K. Saike) was carried out.

Each product (15 $\mu$l) was applied to a polyarylamide gel. A resulting electrophoretic chart was subjected to densitometry. The densitometry showed that concentration of the substance prepared according to the invention was 5 times as great as that of the substance prepared by the conventional technique.

Optimum embodiments of the apparatus for amplification of DNA and certain examples of implementation of the method according to the invention have been described. It will be apparent to those skilled in the art that modifications and improvement of the apparatus may be made without departure from the spirit and scope of the invention. Thus the tubes may be installed on a common base and may detachable connect to one another for replacement by tubes of a different capacity depending on the length of a DNA molecule to be synthesized. Alternatively, a pump may be provided in the closed-loop pipeline for positively pumping the reaction mixture although as temperature difference in the closed-loop pipeline is sufficient for moving the reaction mixture.

Although a vertical arrangement of the closed-loop pipeline has been disclosed for a self-supported movement of a reaction mixture through the pipeline under the action of a temperature difference, the pipeline can be placed horizontally, and in this case a pump must be used to move the reaction mixture.

Certain modifications may be made in the implementation of the method such as changes in heating and cooling rates, lengths of synthesized fragments, etc.

Industrial Applicability

The method for amplification of DNA according to the invention may be used for synthesis of high-molecular DNA on commercial scale.

The method and apparatus according to the invention may be used in molecular biology, gene engineering, medicine, and veterinary for scientific investigations, diagnostic and other purposes.

## Claims

1. A method for amplification of DNA, comprising preparing a reaction mixture consisting of a template DNA, primers of synthetic oligonucleotides, desoxynucleosidetriphosphates, and a buffer medium and carrying out thermocontrolled steps of denaturation of the template DNA, annealing with primers, and completion of the complementary chain, with subsequent repetition of the steps of denaturation, annealing and completion until the process is over, characterized by carrying out the process in a continuous closed-loop system consisting of the following thermocontrolled zones: a DNA fusion zone and a cooling zone ensuring denaturation of the template DNA; a zone for annealing with primers, and a zone for completion of the complementary chain, carrying out circulation of a prepared reaction mixture, which functions as a carrier of DNA molecules which pass during the circulation consecutively through each of said zones to be subjected to denaturation, annealing with primers and completion of the complementary chain, the reaction mixture being circulated until a predetermined amount of DNA is prepared.

2. A method of claim 1, characterized by the fact that the fusion zone has a temperature from 80 to 95°C, the cooling zone has a temperature from 10 to 30°C, the annealing zone has a temperature from 20 to 60°C, and the completion zone has a temperature from 50 to 75°C.

3. A method of any of claims 1-2, characterized by the fact that the circulation is carried out at a heating rate of a reaction mixture from 0.3 to 0.6°C/s with a cooling rate of 8 to 20°C/s.

4. An apparatus for amplification of DNA, characterized by comprising a closed-loop pipeline (1, 2, 3, 4) adapted to be filled with a fluid reaction mixture and by being provided with four thermostats (5, 6, 7, 8) which are mounted downstream one another along the perimeter of the pipeline to define in the pipelines zones with predetermined temperatures corresponding, in the process sequence, to a fusion zone, to a cooling zone, to an annealing zone, and to a zone for completion of the complementary chain.

5. An apparatus of claim 4, characterized by the fact that temperature is maintained in the fusion zone between 80 and 95°C, in the cooling zone between 10 and 30°C, in the annealing zone between 20 and 60°C, and in the zone for completion of the complementary chain between 50 and 75°C.

6. An apparatus of claim 4, characterized by the fact that the pipeline (1, 2, 3, 4) is made up of four series-communicating tubes (1, 2, 3, 4), each tube being placed in respective individual thermostat (5, 6, 7, 8).

7. An apparatus of claim 6, characterized by the fact that the pipeline is made up of capillary tubes (1, 2, 3, 4).

8. An apparatus of claim 7, characterized by the fact that the tubes are installed on a common base and are detachably connected to one another for replacement for varying the capacity in accordance with the length of a DNA molecule to be synthesized.

9. An apparatus of any of claims 4 through 8, characterized by the fact that a reaction mixture flow velocity controlled (15) is provided in one of them portions of the closed-loop-loop pipeline.

10. An apparatus of claim 4, characterized by the fact that the closed-loop-loop pipeline is provided with a pump for positive pumping of a reaction mixture.

11. An apparatus of claim 4, characterized by the fact that the pipeline has a port (16) adapted for provided a liquid stopper, them port being also used for loading a reaction mixture for discharging the end product.

# INTERNATIONAL SEARCH REPORT

International Application No  PCT/SU 91/00200

## I. CLASSIFICATION OF SUBJECT MATTER (If several classification symbols apply, indicate all) ⁶

According to International Patent Classification (IPC) or to both National Classification and IPC

Int.Cl.⁵      C12Q 1/68

## II. FIELDS SEARCHED

### Minimum Documentation Searched ⁷

| Classification System | Classification Symbols |
|---|---|
| Int.Cl.⁵ | C12Q 1/68 |

Documentation Searched other than Minimum Documentation
to the Extent that such Documents are Included in the Fields Searched ⁸

## III. DOCUMENTS CONSIDERED TO BE RELEVANT ⁹

| Category * | Citation of Document, ¹¹ with indication, where appropriate, of the relevant passages ¹² | Relevant to Claim No. ¹³ |
|---|---|---|
| A | EP,A1,0325763 (ORION CORPORATION LTD), 2 August 1989 (02.08.89), the abstract, the claims, figure 1-4 | 4-11 |
| A | EP,A2,0329822 (CANGENE CORPORATION) 30 August 1989 (30.08.89), the abstract, the claims, figure 1 | 1-3 |
| A | EP,A2,0328829 (AMOCO CORPORATION), 23 August 1989 (23.08.89), the abstract, the claims, figure 1a-8 | 1-11 |
| A | US,A,4683195 (CETUS CORPORATION), 28 July 1989 (28.07.87), the abstract, the claims | 1-3 |
| A,P | "Analiz genoma. Metody", pad red. K. Deivisa, 1990, Mir (MOSCOW), pages 178,183 | 1-11 |

* Special categories of cited documents: ¹⁰

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

## IV. CERTIFICATION

| Date of the Actual Completion of the International Search | Date of Mailing of this International Search Report |
|---|---|
| 18 December 1991 (18.12.91) | 16 January 1992 (16.01.92) |

| International Searching Authority | Signature of Authorized Officer |
|---|---|
| ISA/SU | |

Form PCT/ISA/210 (second sheet) (January 1985)